# EUROPEAN PATENT APPLICATION

(11) **EP 4 830 673 A1**
(43) Date of publication of application: **28.10.2026**
(21) Application number: 24912308.4
(22) Date of filing: 09.12.2024
(51) Int. Cl.: A61F 2/852, A61F 2/90

(54) **STENT**

(30) Priority: 25.12.2023 JP 2023218657
(71) Applicant: T.G. Medical Inc., Tokyo 103-0023 (JP)
(72) Inventor: MIKI Kohei, Tokyo 103-0023 (JP)
(74) Representative: Appleyard Lees IP LLP
(86) International application number: PCT/JP2024/043339
(87) International publication number: WO 2025/142419

(57) **Abstract**

Provide is a stent having a two-layer structure capable of appropriately expanding a biological lumen even when the relative position in the circumferential direction between an outer stent body and an inner stent body has shifted. A stent 1 comprises: an outer stent body 10 in which a plurality of outer cells 12 are arranged in the circumferential direction OD and are continuous in the central axis direction LD; and an inner stent body 20 in which a plurality of inner cells 22 are arranged in the circumferential direction and are continuous in the central axis direction LD, the inner stent body 20 being inserted into the outer stent body 10 at least in a region for expanding a biological lumen. The stent 1 has a same shape region A in which cells having the same shape when viewed from the radial direction among the outer cells 12 and the inner cells 22 are arranged adjacent to each other. The same shape region A includes a region in which outer overlapping portions 14 and inner overlapping portions 24 are arranged with the relative positions thereof has shifted in the axial direction of the stent 1.

## Description

### TECHNICAL FIELD

The present disclosure relates to a stent.

### BACKGROUND ART

A catheter treatment in which a stent is deployed in a lesion area in a vascular lumen has been known.

However, in a conventional stent having a single-layer structure as disclosed in Patent Document 1, since its surface area contacting a vascular lumen is small, increasing an expansive force of the stent causes an increase in a pressure with which the stent presses the vascular lumen, leading to an increase in the risk of the blood vessel damage.

Further, as a stent which has a larger surface area and has excellent shape followability to a vascular structure and excellent diameter-narrowing performance, a stent including an outer stent body and an inner stent body to be inserted into the outer stent body (hereinafter, also referred to as a double-layer stent) has been disclosed (see Patent Document 2).

In the stent having a double-layer structure as disclosed in Patent Document 2, since its surface area contacting a vascular lumen is larger than that of the stent having a single-layer structure, the risk of the blood vessel damage can be reduced even when the expansive force of the stent is increased. In the conventional stent having a double-layer structure as disclosed in Patent Document 2, a region where the inner stent body and the outer stent body have the same cell shape occupies most of a region in which the blood vessel or the like is to be expanded. These cells with the same shape are arranged to be offset in the circumferential direction in order to increase the surface area contacting an inner wall of the blood vessel.

### Citation List

### Patent Documents

Patent Document 1: US Patent No. 10390982
Patent Document 2: PCT International Publication No. WO2022/085313

### DISCLOSURE OF THE INVENTION

### Problems to be Solved by the Invention

However, when the positions of the outer stent body and the inner stent body are misaligned relative to each other in the circumferential direction and the outer cell and the inner cell overlap substantially with each other, a mesh opening area formed by the outer stent body and the inner stent body increases. Accordingly, the inner wall of the blood vessel protrudes into the stent, and the stent bodies are embedded into the inner wall of the blood vessel, so that there is a risk that the blood vessel cannot be sufficiently expanded.

A problem of the present disclosure is to provide a stent having a double-layer structure capable of adequately expanding a living body lumen even when positions of an outer stent body and an inner stent body are misaligned relative to each other in a circumferential direction.

### Means for Solving the Problems

The present disclosure addresses the above-described problem by the following solution. Note that for the sake of easy understanding, the present disclosure will be described with reference numerals corresponding to an embodiment, but the disclosure is not limited thereto.

A first aspect of the present disclosure is directed to a stent (1) that is inserted into a catheter and removed from the catheter in a living body lumen to expand the living body lumen, the stent (1) including: an outer stent body (10) in which a plurality of outer cells (12) each surrounded by outer struts (11) and outer overlapping portions (14) at which the outer struts (11) cross each other are arrayed in a circumferential direction and are continuous in a central axis direction; and an inner stent body (20) in which a plurality of inner cells (22) each surrounded by inner struts (21) and inner overlapping portions (24) at which the inner struts (21) cross each other are arrayed in the circumferential direction and are continuous in the central axis direction, and which is inserted into the outer stent body (10) in at least a region that expands the living body lumen, in which the stent (1) has a same-shape region (A) in which cells with a same shape when viewed in a radial direction among the outer cells (12) and the inner cells (22) are arrayed adjacent to each other, and the same-shape region (A) includes a region in which the outer overlapping portions (14) and the inner overlapping portions (24) are positioned to be offset relative to each other in an axial direction of the stent (1).

According to a second aspect of the present disclosure, in the stent (1) of the first aspect, the outer overlapping portions (14) and the inner overlapping portions (24) in the same-shape region (A) are offset from each other by a half of an array pitch with which the outer cells (12) and the inner cells (22) are arrayed in the circumferential direction when viewed in the radial direction.

According to a third aspect of the present disclosure, in the stent (1) of the first or second aspect, each of the outer overlapping portions (14) and each of the inner overlapping portions (24) in the same-shape region (A) are placed to be offset from each other in the axial direction and the circumferential direction of the stent (1) in the outer cells (12) and the inner cells (22).

A fourth aspect of the present disclosure is directed to a method for expanding a living body organ with a lumen structure using a stent (1), the stent (1) including: an outer stent body (10) in which a plurality of outer cells (12) each surrounded by outer struts (11) and outer overlapping portions (14) at which the outer struts (11) cross each other are arrayed in a circumferential direction and are continuous in a central axis direction; and an inner stent body (20) in which a plurality of inner cells (22) each surrounded by inner struts (21) and inner overlapping portions (24) at which the inner struts (21) cross each other are arrayed in the circumferential direction and are continuous in the central axis direction, and which is inserted into the outer stent body (10) in at least a region that expands the living body lumen, in which the stent (1) has a same-shape region (A) in which cells with a same shape when viewed in a radial direction among the outer cells (12) and the inner cells (22) are arrayed adjacent to each other, and the same-shape region (A) includes a region in which the outer overlapping portions (14) and the inner overlapping portions (24) are positioned to be offset relative to each other in an axial direction of the stent (1), the method including: delivering the stent (1, 1B) sheathed in a catheter (50) in a diameter-narrowed state together with the catheter (50), through the living body organ, to a position where the living body organ is to be expanded; retracting the catheter (50) to a proximal side and starting to expand the stent (1, 1B) from a distal side, and completing expanding the stent (1, 1B); and resheathing the expanded stent (1, 1B) in the catheter (50).

### Effects of the Invention

According to the present disclosure, a double-layer stent can be provided which is capable of adequately expanding a living body lumen even when an outer stent body and an inner stent body are misaligned relative to each other in a circumferential direction.

### BRIEF DESCRIPTION OF THE DRAWINGS

FIG. 1 is a side view of a stent 1 of a first embodiment;
FIG. 2A is a virtual development view of a part of an outer stent body 10 of the first embodiment;
FIG. 2B is a virtual development view of a part of an inner stent body 20 of the first embodiment;
FIG. 2C is a virtual development view of a part of the stent 1 of the first embodiment in a state in which a relative position in a circumferential direction between the outer stent body 10 and the inner stent body 20 is in a designed position;
FIG. 3 is a virtual development view of a part of the stent 1 of the first embodiment in a state in which a relative position between the outer stent body 10 and the inner stent body 20 in a circumferential direction is misaligned from a designed position;
FIG. 4A is a diagram for describing an outer diameter D1 of an outer stent body 10 alone;
FIG. 4B is a diagram for describing an outer diameter D2 of an inner stent body 20 alone;
FIG. 5 is a diagram showing a process of inserting the inner stent body 20 into the outer stent body 10;
FIG. 6 is a virtual development view of a part of a stent 1 of a second embodiment in a state in which a relative position in a circumferential direction between an outer stent body 10 and an inner stent body 20 is at a designed position;
FIG. 7 is a virtual development view of a part of the stent 1 of the second embodiment in a state in which a relative position between the outer stent body 10 and the inner stent body 20 in a circumferential direction is misaligned from a designed position;
FIG. 8A is a schematic view showing steps of expanding a narrowed site in a blood vessel using the stent 1;
FIG. 8B is a schematic view showing steps of expanding a narrowed site in a blood vessel using the stent 1;
FIG. 8C is a schematic view showing steps of expanding a narrowed site in a blood vessel using the stent 1; and
FIG. 8D is a schematic view showing steps of expanding a narrowed site in a blood vessel using the stent 1.

### PREFERRED MODE FOR CARRYING OUT THE INVENTION

Hereinafter, embodiments of a stent according to the present disclosure will be described. Note that each of the drawings attached to the present specification shows a schematic view and the shape, scale, longitudinal-lateral dimensional ratio, etc. are changed or exaggerated as compared to actual shape, scale, longitudinal-lateral dimensional ratio, etc. Moreover, in the drawings, hatching showing the cross-section of members may be omitted where appropriate.

In the present specification etc., terms specifying shapes, geometric conditions, and the degrees thereof, such as "perpendicular" and "direction", include not only exact meanings of these terms, but also ranges taken as being substantially perpendicular and being substantially in a direction. In the present specification etc., in an axial direction (a longitudinal direction) LD, a side (a proximal side) close to a practitioner will be referred to as a side LD1 (or a proximal LD1 side), a side (a distal side) distant from the practitioner will be referred to as a side LD2 (or a distal LD2 side), and a direction perpendicular to an axial direction LD will be referred to as a radial direction RD. Moreover, in the present specification etc., a direction in which cells are arrayed will be referred to as a circumferential direction OD.

### (First Embodiment)

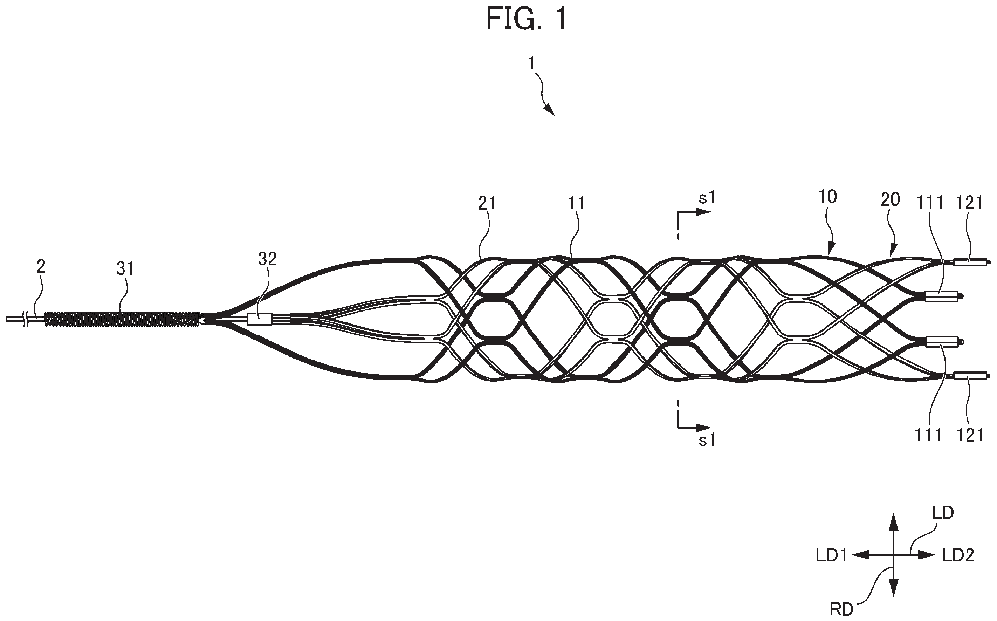

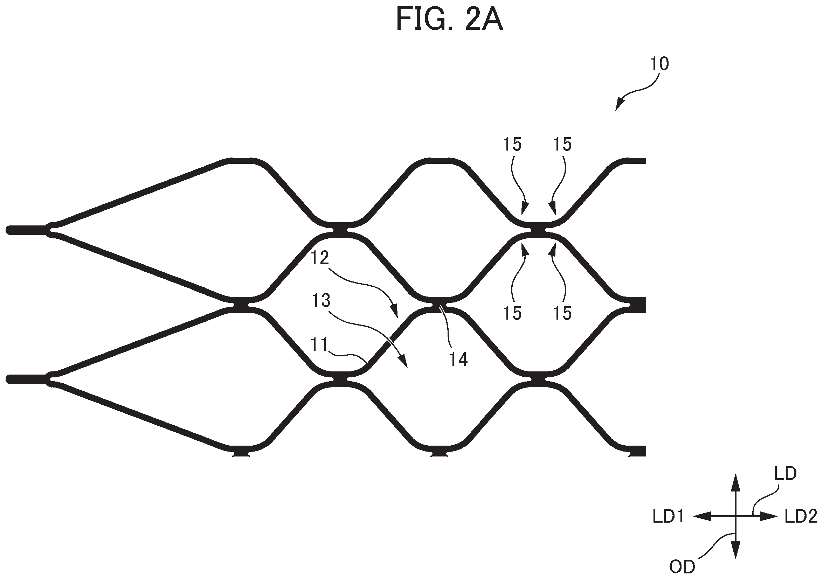

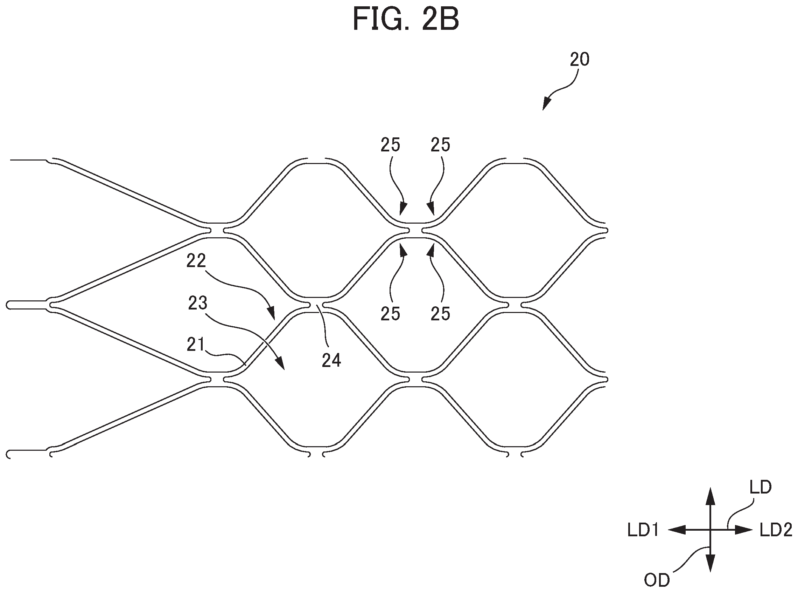

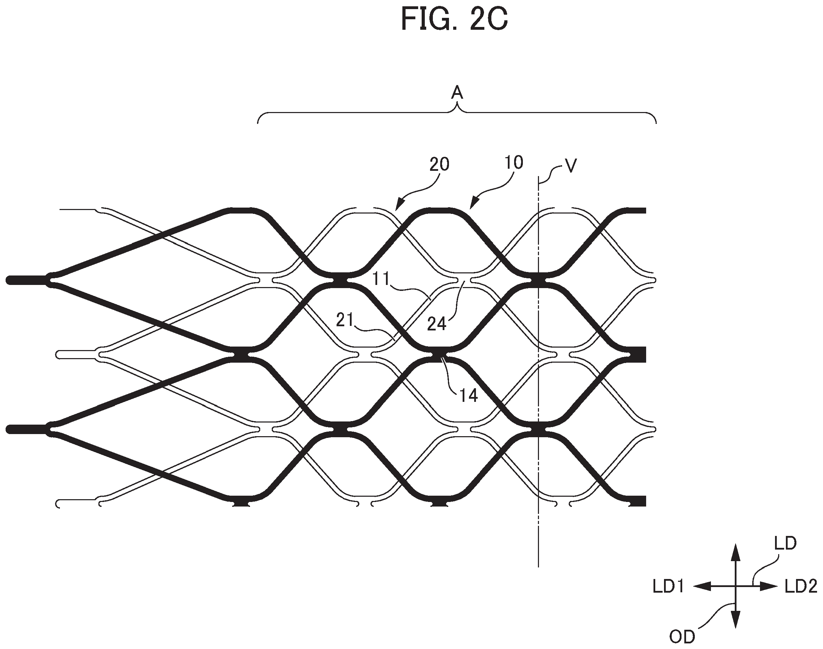

FIG. 1 is a side view of a stent 1 of a first embodiment. FIG. 2A is a virtual development view of a part of an outer stent body 10 of the first embodiment. FIG. 2B is a virtual development view of a part of an inner stent body 20 of the first embodiment. FIG. 2C is a virtual development view of a part of the stent 1 of the first embodiment in a state in which a relative position in a circumferential direction between the outer stent body 10 and the inner stent body 20 is at a designed position.

In the drawings shown in the first embodiment and the other embodiments, for the sake of easy distinguishing of the outer stent body 10 and the inner stent body 20, a strut of the outer stent body 10 is indicated by black, and a strut of the inner stent body 20 is indicated by white. Moreover, in the present specification etc., the "cell" indicates a portion surrounded by a material of a strut (a strut) forming a stent. "Cells" may be identical to each other or different from each other in shape and size. The "strut" indicates a portion made of wire-like material. In the present specification etc., a cell opening will also be referred to as an "opening portion", and a portion where struts of adjacent cells are connected to or overlap with each other will also be referred to as an "overlapping portion".

The stent 1 of the first embodiment may be used, for example, as follows: the stent 1 is sheathed in a catheter and is pushed out of the catheter and is expanded in a vascular lumen, such that the stent 1 expands a narrowed or occluded blood vessel. The stent 1 is used for an application to be retrieved from the body after having been temporarily placed in a blood vessel. Alternatively, the stent 1 may be used while implanted in the blood vessel without being retrieved from the body. When the stent 1 is used for such an application, it may be preferable that a wire 2 and the stent 1 are configured to be separable from each other through the operation by a practitioner.

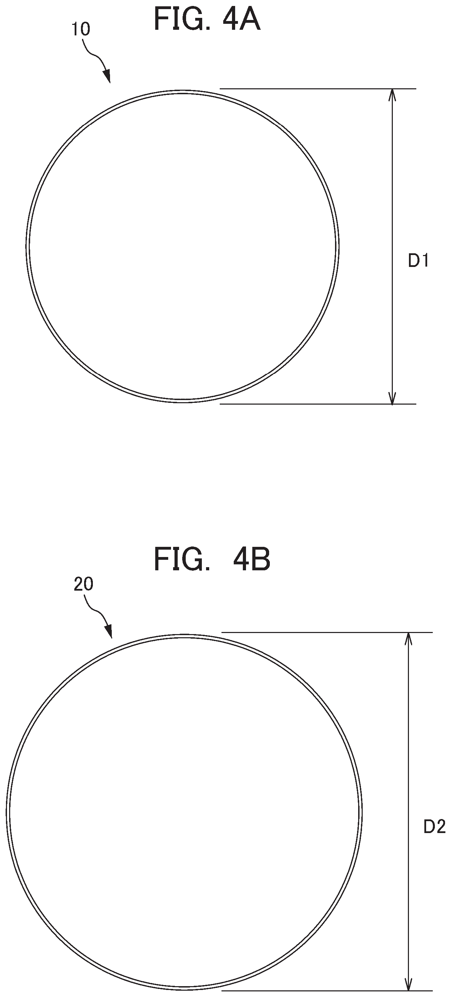

The stent 1 is configured to be substantially cylindrical when the stent 1 is pushed out of the catheter to expand in a free state (see FIGS. 4A and 4B described later). Although not shown, the stent 1 is in an elongated cylindrical shape in a diameter-narrowed state. A wire 2 is connected to a proximal LD1 side end portion of the stent 1. A method for connecting the proximal side end portion of the stent 1 and the wire 2 to each other is not particularly limited as long as the connection method is used for general medical equipment. Examples of the method may include welding, UV bonding, and silver solder infiltration.

The practitioner pushes or pulls the wire 2 via an operation unit (not shown) outside the body that is coupled to the proximal LD1 side of the wire 2, thereby moving the stent 1 back and forth in the catheter or the blood vessel. The practitioner can remove the stent 1 from the catheter and expand the stent 1, or resheathe the stent 1 in the catheter through pulling (moving back) or pushing (moving forth) the catheter without moving the wire 2 (the stent 1).

The stent 1 is a substantially cylindrical structure and includes the outer stent body 10 and the inner stent body 20. The stent 1 is a stent having such a double-layer structure in which the inner stent body 20 is inserted into the outer stent body 10 in at least a region that expands a narrowed site of a living body lumen. Here, the region that expands a narrowed site of a living body lumen refers to a region substantially contributing to expansion of the narrowed site during use. This region corresponds to a region expanding in a substantially cylindrical shape in a diameter-expanded state shown in FIG. 1 in the stent 1 of the present embodiment. Moreover, the above-described "at least" means that in the above-described region that expands a narrowed site, the inner stent body 20 is required to be inserted into the outer stent body 10. Note that the region in which the inner stent body 20 is inserted into the outer stent body 10 is not limited to the above-described region that expands a narrowed site (in the example of the present embodiment, a region expanded in a substantially cylindrical shape). In the present embodiment, the inner stent body 20 is inserted into the outer stent body 10 immediately before the position where the first stent body 10 and the inner stent body 20 are coupled to the wire 2.

The inner stent body 20 being arranged inside (or inserted into) the outer stent body 10 does not indicate that the inner stent body 20 is arranged completely inside the outer stent body 10 at all positions of the inner stent body 20. That is, even in a region in which the inner stent body 20 is arranged inside (inserted into) the outer stent body 10, a part of the inner stent body 20 may be arranged outside an inner surface of the outer stent body 10 in the radial direction or may be arranged outside a surface (an outer surface) of the outer stent body 10. As described later, in the present embodiment, a part of the inner stent body 20 is intentionally arranged outside the inner surface of the outer stent body 10 in the radial direction or arranged outside the surface (the outer surface) of the outer stent body 10. Note that as described above, a part of the outer stent body 10 may be arranged inside a surface (an outer surface) of the inner stent body 20.

The outer stent body 10 and the inner stent body 20 are not coupled to each other in the radial direction. Specifically, the outer stent body 10 and the inner stent body 20 are indirectly connected to each other via the wire 2, but are not coupled to each other in the other regions. Thus, the outer stent body 10 and the inner stent body 20 are independently and flexibly deformable. Also in a diameter-expanded state, the outer stent body 10 and the inner stent body 20 just closely contact each other in the radial direction and are not coupled (connected) to each other in the radial direction. Thus, the outer stent body 10 and the inner stent body 20 are independently deformable, but do not restrict deformation of each other.

The proximal LD1 side of the outer stent body 10, the proximal LD1 side of the inner stent body 20, and the distal LD2 side of the wire 2 are directly connected to each other. Here, "directly connected" refers to, for example, a form in which the proximal LD1 side of the outer stent body 10 and the distal LD2 side of the wire 2 are connected to each other and the proximal LD1 side of the inner stent body 20 and the distal LD2 side of the wire 2 are connected to each other. In this case, a position where the proximal LD1 side of the outer stent body 10 is connected to the wire 2 may be the same as or different from a position where the proximal LD1 side of the inner stent body 20 is connected to the wire 2.

Alternatively, the proximal LD1 side of the outer stent body 10, the proximal LD1 side of the inner stent body 20, and the distal LD2 side of the wire 2 may be indirectly connected to each other. Here, "indirectly connected" refers to, for example, a form in which the proximal LD1 side of the outer stent body 10 and the distal LD2 side of the wire 2 are connected to each other and the proximal LD1 side of the outer stent body 10 and the proximal LD1 side of the inner stent body 20 are connected to each other (or a connection form in which the two stent bodies are connected in an inverse relationship). In this case, the proximal LD1 side of the inner stent body 20 is indirectly connected to the wire 2 via the proximal LD1 side of the outer stent body 10. Alternatively, there may be another form in which the proximal LD1 side of the outer stent body 10 and the proximal LD1 side of the inner stent body 20 are connected to each other via a pipe-like sleeve (not shown) inserted over the distal LD2 side of the wire 2. In this case, the proximal LD1 side of the outer stent body 10 and the proximal LD1 side of the inner stent body 20 are indirectly connected to the distal LD2 side of the wire 2 via the sleeve. As described above, in the present specification, "indirectly connected" includes a form in which one of the proximal LD1 side of the outer stent body 10 and the proximal LD1 side of the inner stent body 20 is directly connected to the wire 2, and the other stent body, which is not connected directly to the wire 2, is indirectly connected to the wire 2 via the stent body directly connected to the wire 2. Examples of a method for connecting the proximal side end portion of each stent body and the wire 2 to each other may include welding, UV bonding, and silver solder infiltration, but is not particularly limited as long as the connection method is used for general medical equipment.

As described later, the stent 1 of the first embodiment is produced in such a manner that the inner stent body 20, which has a greater outer diameter than that of the outer stent body 10 in a natural state, is inserted into the outer stent body 10 in a diameter-narrowed state. Thus, the inner stent body 20 constantly presses the outer stent body 10 outward in the radial direction RD, so that the outer stent body 10 and the inner stent body 20 are closely in contact with each other. As described later, in a diameter-expanded state, a part of the inner stent body 20 can be arranged outside the inner surface of the outer stent body 10 in the radial direction.

As shown in FIG. 1, the proximal LD1 side end portions of the outer stent body 10 and the inner stent body 20, which are tapered and narrowed, are connected to the wire 2. Thus, the inner stent body 20 is restricted from moving in the axial direction relative to the outer stent body 10.

The stent 1 includes, as markers, a first wire marker 31, a second wire marker 32, an outer stent marker 111, and an inner stent marker 121. These markers each are a member serving as a mark for checking the position and the state of the stent 1 under observation by radiation in the tubular organ such as a blood vessel, and are formed of a material which is highly opaque to radiation (has a high radiopacity). Here, the radiation opacity indicates that, under observation using radiation during operation, the markers are more highly opaque to radiation than portions other than the markers in the stent 1 (other portions having no radiation opacity), and are opaque to radiation to the extent that the markers can be observed as images. The "radiation opacity" is required to obtain the "visibility" and "radiopacity" under observation using radiation.

As a highly radiopaque material, which can be used as a marker, a metal or a synthetic resin may be used, and examples of such a metal material may include gold, tantalum, platinum, tungsten, iridium, and platinum tungsten, and an alloy containing these materials. Moreover, examples of such a material may include a polymer material having radiation opacity to which a radiopaque filler or the like is added. When the marker is formed of a linear member, a wire made of a composite member having a coaxial core material made of the above-described metal material in a wire made of nickel titanium can be used, for example.

As a method of attaching the markers to the stent 1, a conventional known manufacturing method used when the markers are attached to the stent, such as gold-tin solder, silver-tin solder, laser welding, mechanical pressing, bonding with resin, can be used where appropriate.

The first wire marker 31 is a coil marker which is formed of a highly radiopaque material into a coil shape, , and is fixed to a position covering the proximal LD1 side end portion of the outer strut 11 by welding. The second wire marker 32 is a ring marker in which a marker component, which is made of a highly radiopaque material and formed in a circular tube shape is fixed to a strut by soldering. The second wire marker 32 is fixed to a position covering the proximal LD1 side end portion of the inner stent body 20 by soldering. The outer stent marker 111 is a crimping marker which is formed in a substantially square pipe shape, and is crimp-fixed to the distal LD2 side end portion of the outer stent body 10. The inner stent marker 121 is a crimping marker which is formed in a substantially square pipe shape, and is crimp-fixed to the distal LD2 side end portion of the inner stent body 20 in a form similar to the outer stent marker 111. Note that the first wire marker 31, the second wire marker 32, the outer stent marker 111, and the inner stent marker 121 in the present embodiment are exemplified as parts of specific forms of the markers, and the locations and the number of the markers can be changed where appropriate.

As shown in FIG. 2A, in the outer stent body 10, a plurality of outer cells 12 including the outer struts 11 arranged to surround regions except for cells on the proximal LD1 side and the distal LD2 side in a substantially rhombus shape are arrayed in the circumferential direction OD. In the outer stent body 10, the plurality of outer cells 12 arrayed in the circumferential direction OD are arrayed in the axial direction LD. That is, the outer stent body 10 has such a mesh pattern that the plurality of outer cells 12 are arrayed in the circumferential direction OD and the axial direction LD. An opening portion 13 is formed in the outer cell 12. Moreover, the outer cells 12 adjacent to each other in the circumferential direction OD are connected to each other at an outer overlapping portion 14. As described above, the proximal LD1 side of the outer stent body 10 gradually converges toward the wire 2, and is connected to the wire 2. In the present embodiment, the distal LD2 side of the outer stent body 10 converges toward the two outer stent markers 111.

The outer overlapping portion 14 is a portion to which the outer struts 11 for the four outer cells 12 adjacent to each other are connected. The outer overlapping portion 14 has a substantially rectangular shape elongated in the axial direction LD. The outer struts 11 are individually connected to four corners of the outer overlapping portion 14. In each of the outer struts 11, a curved portion 15 is formed at a portion connected to the outer overlapping portion 14. Thus, when the expanded stent 1 is bent into a substantially U shape, each of the outer struts 11 connected to the outer overlapping portion 14 is independently deformable. Therefore, the outer cells 12 arrayed in the circumferential direction OD can be more flexibly bent. As described above, the outer cells 12 arrayed in the circumferential direction OD can be flexibly bent, and therefore, the outer stent body 10 is excellent in shape followability and diameter-narrowing performance.

As shown in FIG. 2B, in the inner stent body 20, a plurality of inner cells 22 including the inner struts 21 arranged to surround regions except for cells on the proximal LD1 side and the distal LD2 side in a substantially rhombus shape are arrayed in the circumferential direction OD. In the inner stent body 20, the plurality of inner cells 22 arrayed in the circumferential direction OD are arrayed in the axial direction LD. That is, the inner stent body 20 has such a mesh pattern that the plurality of inner cells 22 are arrayed in the circumferential direction OD and the axial direction LD. An opening portion 23 is formed in the inner cell 22. Moreover, the inner cells 22 adjacent to each other in the circumferential direction OD are connected to each other at an inner overlapping portion 24. As described above, the proximal LD1 side of the inner stent body 20 gradually converges toward the wire 2, and is connected to the wire 2. In the present embodiment, the distal LD2 side of the inner stent body 20 converges toward the two inner stent markers 121.

The inner overlapping portion 24 is a portion to which the inner struts 21 for the four inner cells 22 adjacent to each other are connected. The inner overlapping portion 24 has a substantially rectangular shape elongated in the axial direction LD. The inner struts 21 are individually connected to four corners of the inner overlapping portion 24. In each of the inner struts 21, a curved portion 25 is formed at a portion connected to the inner overlapping portion 24. Thus, when the expanded stent 1 is bent into a substantially U shape, each of the inner struts 21 connected to the inner overlapping portion 24 is independently deformable. Therefore, the inner cells 22 arrayed in the circumferential direction OD can be more flexibly bent. As described above, the inner cells 22 arrayed in the circumferential direction OD can be flexibly bent, and therefore, the inner stent body 20 is excellent in shape followability and diameter-narrowing performance.

As shown in FIG. 2C, the stent 1 of the first embodiment has a same-shape region A in which cells with a same shape (the same size and the same shape) when viewed in the radial direction are arrayed adjacent to each other for the outer cells 12 and the inner cells 22 except for cells on the proximal LD1 side and the distal LD2 side. That is, the mesh pattern of the outer stent body 10 shown in FIG. 2A and the mesh pattern of the inner stent body 20 shown in FIG. 2B are substantially the same pattern in a range of the same-shape region A except for regions on the proximal LD1 side and the distal LD2 side. Here, meaning of the cells with the same shape (the same size and the same shape) is to have substantially the same pattern. And it includes a case where there are manufacturing error and minor differences in dimension and shape.

As shown in FIG. 2C, in the stent 1, the outer stent body 10 and the inner stent body 20 are overlapped such that the inner overlapping portion 24 of the inner cell 22 (the inner stent body 20) is arranged in the opening portion 13 of the outer cell 12 (the outer stent body 10). Specifically, in the same-shape region A, the inner overlapping portion 24 of the inner cell 22 is arranged in the opening portion 13 of the outer cell 12 and the outer overlapping portion 14 of the outer cell 12 is arranged in the opening portion 23 of the inner cell 22. Outer overlapping portions 14 and inner overlapping portions 24 in the same-shape region A are offset from each other by a half of an array pitch with which the outer cells 12 and the inner cells 22 are arrayed in the circumferential direction OD when viewed in the radial direction. Consequently, the inner overlapping portion 24 is arranged to face the opening portion 13 of the outer cell 12, and the outer overlapping portion 14 is arranged to face the opening portion 23 of the inner cell 22. Four outer struts 11 and four inner struts 21 converge on the outer overlapping portion 14 and the inner overlapping portion 24, respectively. Thus, the above-described arrangement enables the struts 21 and the struts 11 to contact the inner wall of the blood vessel in the opening portion 13 and the opening portion 23, respectively. Here, the array pitch with which the outer cells 12 and the inner cells 22 are arrayed in the circumferential direction OD corresponds to a width of one cell (the outer cell 12 or the inner cell 22) in the circumferential direction OD.

In the same-shape region A, positions of the outer overlapping portion 14 of the outer cell 12 and the inner overlapping portion 24 of the inner cell 22 are offset relative to each other in the axial direction LD of the stent 1. That is, the inner overlapping portion 24 is arranged not to overlap with a straight line V drawn virtually on the outer overlapping portion 14 along the circumferential direction OD.

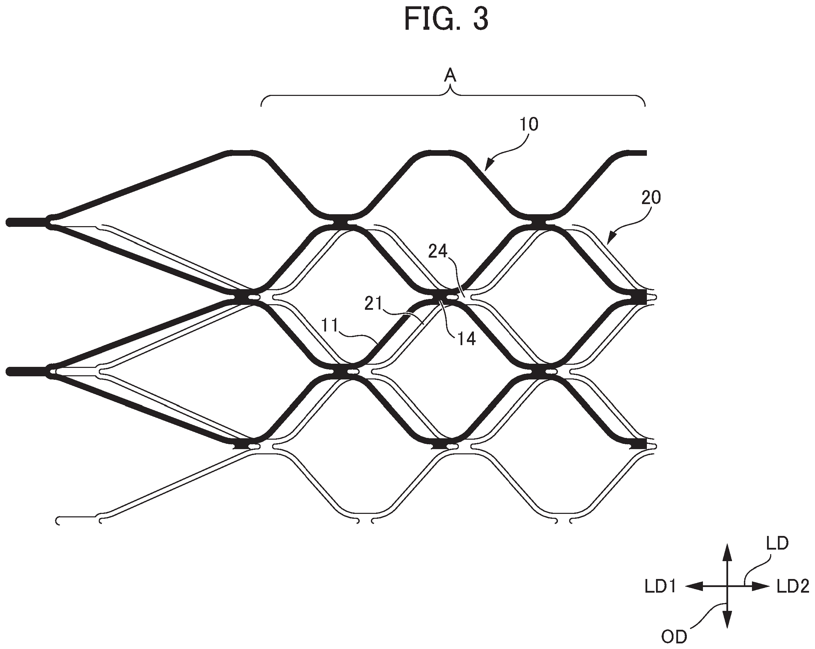

FIG. 3 is a development view showing a state in which a part of the stent 1 of the first embodiment is virtually opened in a planar shape in a state in which a relative position between the outer stent body 10 and the inner stent body 20 in the circumferential direction is misaligned from a designed position. In the example shown in FIG. 3, a case is shown where the positions of the outer stent body 10 and the inner stent body 20 are misaligned relative to each other in the circumferential direction up to a position where the outer overlapping portion 14 comes closest to the inner overlapping portion 24. The offset amount in FIG. 3 corresponds to a half of the array pitch with which the outer cells 12 and the inner cells 22 are arrayed in the circumferential direction OD. As shown in FIG. 3, in the stent 1 of the present embodiment, even in the same-shape region A in which the outer cells 12 and the inner cells 22 have substantially the same shape, the outer stent body 10 and the inner stent body 20 do not overlap completely with each other when the positions of the outer stent body 10 and the inner stent body 20 are misaligned relative to each other in the circumferential direction. Consequently, a mesh opening area formed by the outer stent body 10 and the inner stent body 20 can be prevented from being increased, so that the living body lumen can be adequately expanded. Moreover, both of the outer struts 11 and the inner struts 21 can contact the inner wall of the living body lumen such as the inner wall of the blood vessel. Accordingly, it is possible to control an increase in an expansive pressure when the stent 1 expands the inner wall of the blood vessel, etc. Since the positions of the outer overlapping portions 14 and the inner overlapping portions 24 are offset relative to each other in the axial direction LD, the stent 1 ensures high safety even in the case where the positions of the outer stent body 10 and the inner stent body 20 are misaligned relative to each other in the circumferential direction.

Moreover, the outer overlapping portions 14 and the inner overlapping portions 24 are offset from each other by a half of an array pitch with which the outer cells 12 and the inner cells 22 are arrayed in the circumferential direction OD when viewed in the radial direction. Consequently, the offset amount in the circumferential direction OD up to a position where the outer overlapping portion 14 comes closest to the inner overlapping portion 24 as shown in FIG. 3 can be equalized in both directions with respect to the circumferential direction OD. Accordingly, an ideal arrangement is provided for the unpredictable relative positional misalignment between the outer stent body 10 and the inner stent body 20 in the circumferential direction.

Moreover, the outer overlapping portion 14 and the inner overlapping portion 24 have wider width than the outer strut 11 and the inner strut 21. Accordingly, the outer diameter in a state in which the stent 1 is most narrowed is determined by the outer diameter of a portion where the outer overlapping portion 14 and the inner overlapping portion 24 are arranged. In the stent 1 of the present embodiment, since the positions of the outer overlapping portion 14 and the inner overlapping portion 24 are offset relative to each other in the axial direction LD of the stent 1, the outer overlapping portion 14 and the inner overlapping portion 24 do not overlap with each other at the same position in the diameter-narrowed state of the stent 1. Consequently, the stent 1 of the present embodiment allows its outer diameter when the stent 1 is most narrowed to be smaller than the conventional stent in which the outer overlapping portion 14 and the inner overlapping portion 24 overlap with each other at the same position in the diameter-narrowed state. Accordingly, the stent 1 is readily accommodated in a catheter or the like.

The outer overlapping portion 14 and the inner overlapping portion 24 have wider width than the outer strut 11 and the inner strut 21. Accordingly, they have higher stiffness than the outer strut 11 and the inner strut 21. Consequently, when the outer overlapping portion 14 and the inner overlapping portion 24 overlap with each other in the axial direction LD, the stiffness locally increases. Accordingly, the bendability over the entire stent 1 is deteriorated, which may result in a risk that it is difficult for the stent 1 to pass through the bending portion such as a blood vessel using the catheter. However, in the stent 1 of the present embodiment, the positions of the outer overlapping portions 14 and the inner overlapping portions 24 are offset relative to each other in the axial direction LD of the stent 1. Accordingly, it is possible to prevent the stiffness from locally increasing, achieve good bendability over the entire stent 1, and exhibit excellent shape followability.

Overlapping the mesh patterns of the stent bodies with each other as described above allows the density of the mesh pattern to increase over the entire stent. Thus, the surface area of the stent 1 can be increased. The stent 1 of the present embodiment is configured such that the outer overlapping portion 14 of the outer cell 12 has the configuration shown in FIG. 2A and the inner overlapping portion 24 of the inner cell 22 has the configuration shown in FIG. 2B. Thus, the stent 1 has excellent shape followability and diameter-narrowing performance. Moreover, since the stent 1 is configured such that the outer cells 12 and the inner cells 22 have the configuration described above, the stent 1 produces effects that the narrowed stent 1 is easily sheathed in a catheter and the stent 1 expanded in a blood vessel is easily resheathed in a catheter.

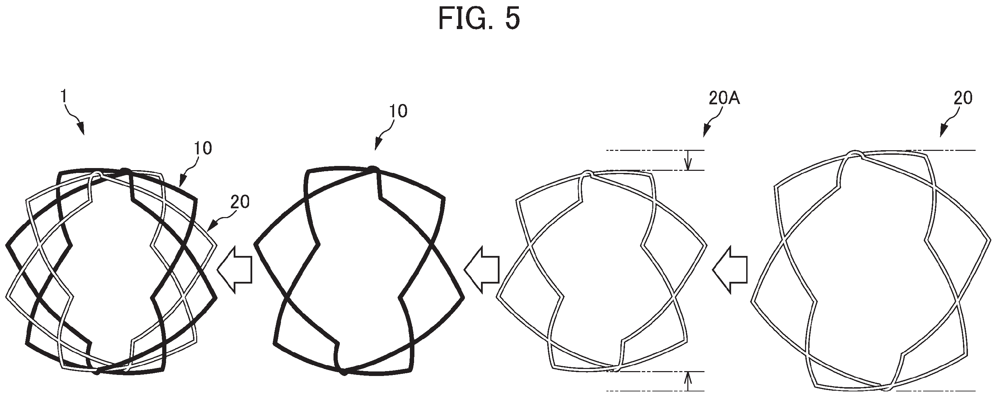

FIG. 4A is a diagram for describing an outer diameter D1 of the outer stent body 10 before assembly. FIG. 4B is a diagram for describing an outer diameter D2 of the inner stent body 20 before assembly. FIG. 5 is a diagram showing a process of inserting the inner stent body 20 into the outer stent body 10. As shown in FIGS. 4A and 4B, in the first embodiment, a relationship between the outer diameter D1 of an outer stent body 10 alone and the outer diameter D2 of an inner stent body 20 alone is set to D1 < D2. Thus, as shown in FIG. 5, an inner stent body 20A obtained by narrowing the inner stent body 20 is inserted into the outer stent body 10. In this manner, due to expansive force of the inner stent body 20 itself, the inner stent body 20 closely contacts inside the outer stent body 10 in the radial direction RD. Thus, in the axial direction LD (see FIG. 1) of the stent 1, it is less likely that the positions of the outer stent body 10 and the inner stent body 20 are misaligned relative to each other. Note that for the sake of easy understanding, FIG. 4 shows only an annular circumferential cell line of each stent body. For the sake of easy understanding of the tubular form, FIG. 4 shows the increased number of cells as compared to that in the other figures and the simplified shape of the cell.

On the other hand, in the circumferential direction around the axial direction LD (see FIG. 1) of the stent 1, there may be a case where the positions of the outer stent body 10 and the inner stent body 20 are misaligned relative to each other. However, as described above, in the stent 1 of the present embodiment, the positions of the outer overlapping portions 14 and the inner overlapping portions 24 are offset relative to each other in the axial direction LD of the stent 1. Consequently, a living body lumen can be adequately expanded even when the positions of the outer stent body 10 and the inner stent body 20 are misaligned relative to each other in a circumferential direction. Moreover, since it is possible to prevent the surface area of the stent 1 which contacts the inner wall of the blood vessel from decreasing, the safety can be enhanced.

The stent 1 of the above-described first embodiment is deployed in a lesion area in a vascular lumen, and in this manner, the vascular lumen can be expanded, and the patency of the lesion area can be ensured. When the stent 1 is removed (retrieved) from the vascular lumen after a predetermined period of time, it is possible to prevent the occurrence of restenosis or reocclusion due to the placed stent or a complication such as a thrombosis. Since the stent 1 of the first embodiment has excellent shape followability, the vascular preservation is high. Note that the stent 1 of the first embodiment is not limited to use for the blood vessel, and for example, may also be used generally for lumen structures, for example, treatment of the esophagus or the large intestine.

The stent 1 of the first embodiment can also be used for treatment of a cerebrovascular spasm. Note that stents of other embodiments to be described later also produce advantageous effects similar to those of the stent 1 of the first embodiment.

Moreover, the stent 1 of the first embodiment can be placed in the blood vessel for a predetermined period of time or implanted permanently. Examples of a stent which is implanted in the blood vessel include a stent for treating an aneurysm. The stent for treating an aneurysm is deployed and implanted at a target position. The purpose of implanting the stent for treating an aneurysm at the target position is, for example, to reduce the blood flowing into the aneurysm existing in the target position and to hold an embolic coil implanted in the aneurysm. After the stent for treating an aneurysm is implanted at the target position, the embolic coil (not shown) is delivered to the distal LD2 side via a microcatheter (not shown) inserted into the catheter, so that the embolic coil can be implanted in the aneurysm through spaces in the mesh of the stent for treating an aneurysm.

Examples of a stent which is used for being implanted in the blood vessel include a stent for being implanted in a cerebral blood vessel, and the stent 1 of the first embodiment can be used for treating a cerebrovascular disease. The cerebrovascular disease mainly includes an ischemic cerebrovascular disease and a hemorrhagic cerebrovascular disease. Examples of the ischemic cerebrovascular disease include cerebral infarction such as atherothrombotic cerebral infarction, lacunar infarction, and cardiogenic cerebral embolism. Examples of the hemorrhagic cerebrovascular disease include a cerebral hemorrhage and a subarachnoid hemorrhage.

### (Second Embodiment)

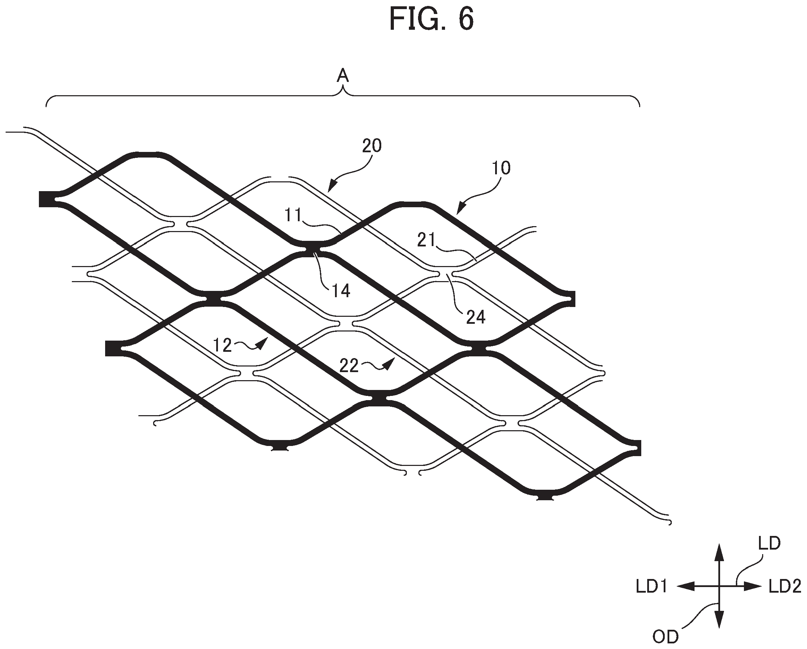

FIG. 6 is a development view showing a state in which a part of a stent 1 of a second embodiment is virtually opened in a planar shape in a state in which a relative position between an outer stent body 10 and an inner stent body 20 in the circumferential direction is at a designed position. The stent 1 of the second embodiment is similar to the stent 1 of the first embodiment except that outer cells 12 and inner cells 22 have a substantially parallelogram shape. Thus, the description of the similar portions to those in the first embodiment will be omitted.
Four corners of adjacent outer cells 12 having a substantially parallelogram shape are arrayed adjacent to each other at an outer overlapping portion 14 in the circumferential direction OD and the axial direction LD. Four corners of adjacent inner cells 12 having a substantially parallelogram shape are arrayed adjacent to each other at an inner overlapping portion 24 in the circumferential direction OD and the axial direction LD.

Similarly to the stent 1 of the first embodiment, in the stent 1 of the second embodiment, outer overlapping portions 14 and inner overlapping portions 24 are positioned to be offset relative to each other in the axial direction LD of the stent 1. Moreover, in the stent 1 of the second embodiment, the outer overlapping portions 14 and the inner overlapping portions 24 are positioned to be offset relative to each other also in the circumferential direction OD of the stent 1. Further, similarly to the stent 1 of the first embodiment, the stent 1 has a same-shape region A in which cells with a same shape when viewed in the radial direction among the outer cells 12 and the inner cells 22 except for cells on the proximal LD1 side and the distal LD2 side are arrayed adjacent to each other.

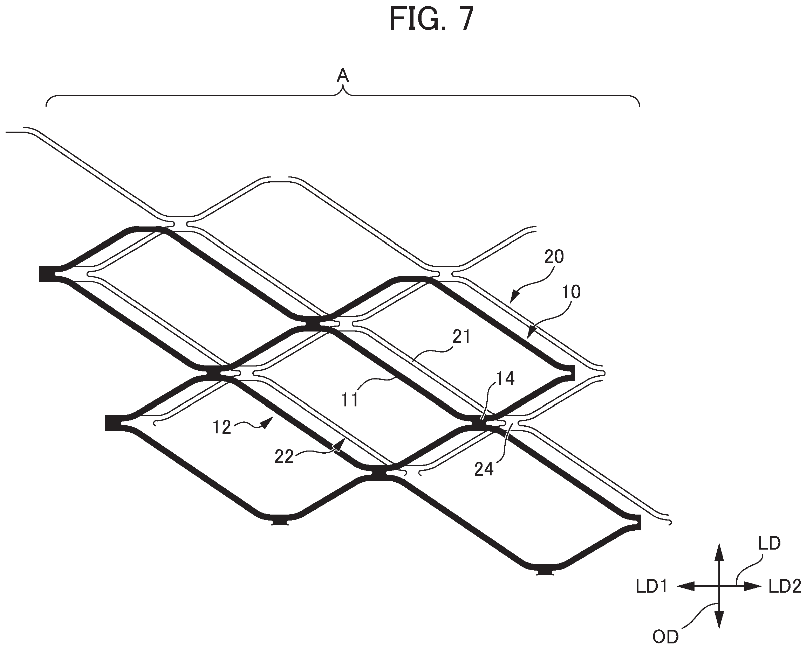

FIG. 7 is a development view showing a state in which a part of the stent 1 of a second embodiment is virtually opened in a planar shape in a state in which a relative position between the outer stent body 10 and the inner stent body 20 in a circumferential direction is misaligned from a designed position. In the example shown in FIG. 7, a case is shown where the positions of the outer stent body 10 and the inner stent body 20 are relatively misaligned from each other in the circumferential direction up to a position where the outer overlapping portion 14 comes closest to the inner overlapping portion 24. In the stent 1 of the second embodiment, the outer overlapping portions 14 and the inner overlapping portions 24 in the same-shape region A are offset from each other in the axial direction LD and the circumferential direction OD of the stent 1 in the outer cells 12 and the inner cells 22, respectively. Thus, as shown in FIG. 7, in the stent 1 of the present embodiment, even in the same-shape region A in which the outer cells 12 and the inner cells 22 have substantially the same shape, the outer stent body 10 and the inner stent body 20 do not overlap completely when the positions of the outer stent body 10 and the inner stent body 20 are misaligned relative to each other in the circumferential direction. Consequently, both of outer struts 11 and inner struts 21 can contact the inner wall of the living body lumen such as the inner wall of the blood vessel, and an expansive pressure when the stent 1 expands the inner wall of the blood vessel, etc. can be prevented from extremely increasing. Thus, since the outer overlapping portions 14 and the inner overlapping portions 24 are arranged to be offset relative to each other in the axial direction LD, the stent body 1 can adequately expand the living body lumen and ensure high safety, even in the case where the outer stent body 10 and the inner stent body 20 are misaligned relative to each other in the circumferential direction.

### (Method of Use)

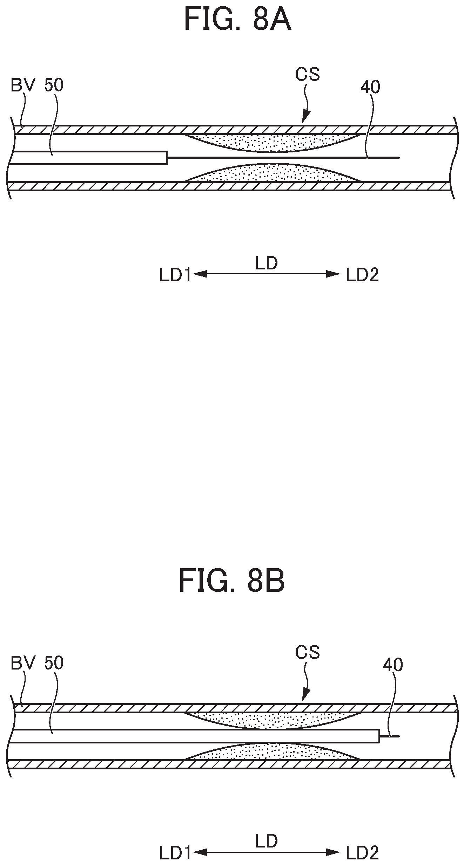

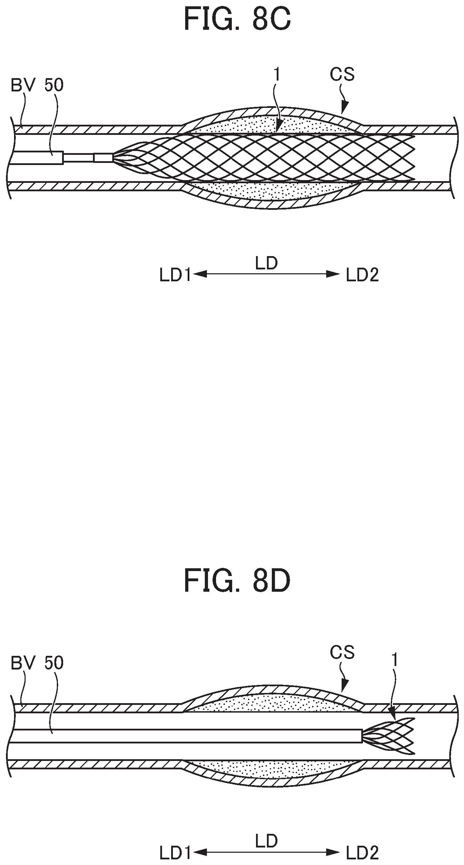

Next, an example of a method for using the stent 1 of each embodiment will be described. FIGS. 8A to 8D each are a schematic view showing steps of expanding a narrowed site in a blood vessel using the stent 1. In FIGS. 8C and 8D, the shape of the stent 1 is simplified. Here, an operation for expanding a narrowed site CS formed in a blood vessel BV to ensure the blood flow will be described. Note that the following operation is performed while grasping a position of the stent or the like using a visibility marker (a radiopaque marker).

First, as shown in FIG. 8A, a guide wire 40 is passed through the narrowed site CS. A catheter 50 is inserted over the guide wire 40. Next, as shown in FIG. 8B, a distal end of the catheter 50 inserted over the guide wire 40 is inserted into the narrowed site CS along the passed guide wire 40, and is passed to reach the distal LD2 side of the narrowed site CS. Next, although not shown in the figure, the guide wire 40 is drawn to retrieve from the body through the catheter 50. Then, the stent 1 is inserted from the proximal LD1 side of the catheter 50. The stent 1 is inserted into the catheter 50 in a diameter-narrowed state.

Next, as shown in FIG. 8C, the stent 1 is deployed from the distal end of the catheter 50 in the distal LD2 side of the narrowed site CS. The distal end of the catheter 50 is located at the distal LD2 side of the narrowed site CS and the catheter 50 is drawn to the proximal LD1 side, so that the stent 1 can be deployed from the distal end of the catheter 50. When the stent 1 is deployed from the distal end of the catheter 50, the stent 1 is expanded by a self-expanding force, so that the narrowed site CS is expanded from the inside. The narrowed site CS is expanded from the inside by the expanded stent 1, so that the blood flow in the blood vessel BV can be ensured.

After it is checked that the blood flow in the blood vessel BV has been ensured, for example, observation of about 5 to 60 minutes is performed. After the observation, as shown in FIG. 8D, the distal end of the catheter 50 is advanced to the distal LD2 side of the stent 1, and the entire stent 1 is resheathed in the catheter 50. Thus, the stent 1 can be retrieved from the body together with the catheter 50.

### (Modification)

The present disclosure is not limited to the above-described embodiments and various modifications and changes can be made, and these modifications and changes are also included in the scope of the present disclosure.

For example, in each embodiment, examples have been described above in which the outer cells 12 and the inner cells 22 have a substantially rhombus shape or a substantially parallelogram shape. The present disclosure is not limited thereto. Alternatively, the shape of the outer cells 12 and the inner cells 22 may be a substantially hexagonal shape and changed as necessary.

Note that each embodiment and modification may be combined as necessary, but detailed description thereof will be omitted. The present disclosure is not limited to each embodiment described above.

### EXPLANATION OF REFERENCE NUMERALS

1 Stent
2 Wire
10 Outer stent body
11 Outer strut
12 Outer cell
13 Opening portion
14 Outer overlapping portion
15 Curved portion
20 Inner stent body
20A Inner stent body
21 Inner strut
22 Inner cell
23 Opening portion
24 Inner overlapping portion
25 Curved portion
31 First wire marker
32 Second wire marker
111 Outer stent marker
121 Inner stent marker

## Claims

1. A stent that is inserted into a catheter and removed from the catheter in a living body lumen to expand the living body lumen, the stent comprising:
an outer stent body in which a plurality of outer cells each surrounded by outer struts and outer overlapping portions at which the outer struts cross each other are arrayed in a circumferential direction and are continuous in a central axis direction; and
an inner stent body in which a plurality of inner cells each surrounded by inner struts and inner overlapping portions at which the inner struts cross each other are arrayed in the circumferential direction and are continuous in the central axis direction, and which is inserted into the outer stent body in at least a region that expands the living body lumen, wherein
the stent has a same-shape region in which cells with a same shape when viewed in a radial direction among the outer cells and the inner cells are arrayed adjacent to each other, and
the same-shape region includes a region in which the outer overlapping portions and the inner overlapping portions are positioned to be offset relative to each other in an axial direction of the stent.

2. The stent according to claim 1, wherein
the outer overlapping portions and the inner overlapping portions in the same-shape region are offset from each other by a half of an array pitch with which the outer cells and the inner cells are arrayed in the circumferential direction when viewed in the radial direction.

3. The stent according to claim 1 or 2, wherein
each of the outer overlapping portions and each of the inner overlapping portions in the same-shape region are placed to be offset from each other in the axial direction and the circumferential direction of the stent in the outer cells and the inner cells.
